# EUROPEAN PATENT APPLICATION

(11) **EP 2 025 733 A1**
(43) Date of publication of application: **18.02.2009**
(21) Application number: 07739000.3
(22) Date of filing: 19.03.2007
(51) Int. Cl.: C09K 11/06, C07D 513/04, H01L 51/50

(54) **MATERIAL FOR ORGANIC ELECTROLUMINESCENCE ELEMENT, AND ORGANIC ELECTROLUMINESCENCE ELEMENT**

(30) Priority: 27.04.2006 JP 2006122936; 27.12.2006 JP 2006350785
(71) Applicant: TOYO INK MFG. CO., LTD., Tokyo 104-0031 (JP)
(72) Inventor: TAKATA, Yoshiyuki c/o Toyo Ink Mfg Co.,Ltd, Tokyo 104-0031 (JP)
(74) Representative: Bauch-Koepe, Katharina Anna
(86) International application number: PCT/JP2007/055557
(87) International publication number: WO 2007/125692

(57) **Abstract**

An object of the present invention is to provide a material for high-brightness, high-efficiency, longer-life organic EL elements and an organic EL element produced by using the same.

Provided is a material for organic EL elements, comprising a compound represented by the following General Formula (1): 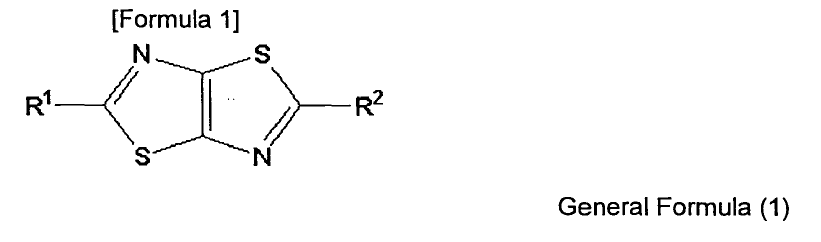 wherein, R¹ and R² each independently represent a substituted or unsubstituted alkyl group, a substituted or unsubstituted aryl group or a substituted or unsubstituted heterocyclic group.

## Description

### TECHNICAL FIELD

The present invention relates to a material for organic electroluminescent elements for planar light source and display devices and an organic electroluminescent element produced by using the same.

### BACKGROUND ART

In organic electroluminescent elements (hereinafter, referred to as organic EL elements), light emission occurs by recombination between electrons injected from cathode and positive holes injected from anode in an organic fluorescent substance layer held between the electrodes. Organic EL elements have attracted attention as a solid emission-type display device, and intensive research and development are under progress recently.

These studies originate from the organic thin film-laminated EL element developed by C. W. Tang and colleagues in Eastman Kodak. According to their research report, an green-light-emitting organic EL element having a brightness of several thousands (cd/m²) at a DC voltage of 6 to 10 V and a maximum luminous efficiency of 1.5 (lm/W) was obtained by using a metal chelate complex as the light-emitting layer and an amine compound as the hole-injecting layer. Currently, studies aimed at improving the efficiency and durability of organic EL elements are in progress for commercialization of full-color display devices in various research organizations. For that reason, materials in various structures have been studied as the materials for organic EL elements.

Organic EL elements produced by using a heterocyclic compound were studied, and, for example, an organic EL element employing a pyrazine derivative as the light-emitting material showed yellow to orange emission (Patent Document 1). Alternatively, an organic EL element produced by using a benzoxazole or thiazole derivative as the light-emitting material showed blue emission (Patent Documents 2, 3 and 4). In addition to use as a light-emitting material, use of a diazole derivative as an electron-transporting material is also studied (Patent Document 5).

For improvement in properties of organic EL elements, a method of forming a light-emitting layer by doping a light-emitting layer host material with a trace of dopant for example by codeposition and thus obtaining emission of the dopant has been studied as an effective means. For example, organic EL elements emitting orange to red light that are prepared by using tris(8-quinolinolato)aluminum as the host material and a 4H-pyran derivative such as DCM, DCJ, DCJT, or DCJTB as the dopant are reported (Non-Patent Document 2).

However, these organic EL elements are still unsatisfactory in properties such as efficiency, operating voltage and lifetime. Thus, there exist a need for development of a material that gives such a high-efficiency, low-operating voltage, and longer-life organic EL element.
Non-Patent Document 1: Appl. Phys. Lett., 51, p.913, 1987
Non-Patent Document 2: Macromol. Symp., 125, p. 1 to 48, and p. 49 to 58, 1997
Patent Document 1: Japanese Patent Application Laid-Open No. 5-178842
Patent Document 2: Japanese Patent Application Laid-Open No. 11-29556
Patent Document 3: Japanese Patent Application Laid-Open No. 11-283746
Patent Document 4: Japanese Patent Application Laid-Open No. 2000-103786
Patent Document 5: Japanese Patent Application Laid-Open No. 2001-288172

### DISCLOSURE OF THE INVENTION

### Problems to be Solved by the Invention

An object of the present invention is to provide a material giving a high-brightness, high-efficiency and longer-lifetime organic EL elements and an organic EL element produced by using the same.

### Means for Solving the Problems

An aspect of the present invention relates to a material for organic EL elements, comprising a compound represented by the following General Formula (1). The material for organic EL elements may consist of the compound represented by the following General Formula (1):

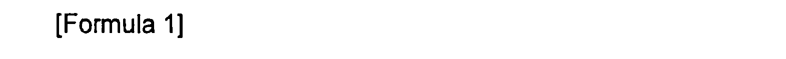

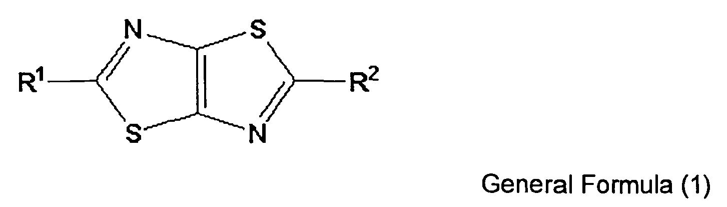

wherein, R¹ and R² each independently represent a substituted or unsubstituted alkyl group, a substituted or unsubstituted aryl group or a substituted or unsubstituted heterocyclic group.

Preferably, R¹ and R² each independently represent a substituted or unsubstituted aryl group having rings of 6 to 30 carbon atoms or a substituted or unsubstituted heterocyclic group having rings of 3 to 30 carbon atoms and 1 to 10 heteroatoms.

The material for organic EL elements may be used as a light-emitting layer material for organic EL elements.

The material for organic EL elements may be used as a light-emitting material for organic EL elements.

The material for organic EL elements may be used as an electron-injecting/transporting material for organic electroluminescent elements.

Another aspect of present invention relates to an organic EL element prepared by using the material for organic EL elements.
The disclosure of the present description, which is related to the subjects of Japanese Patent Application No. 2006-122936 (filed on April 27, 2006) and Japanese Patent Application No. 2006-350785 (file on Dec. 27, 2006) is incorporated herein by reference.

### Effects of the invention

The compound represented by General Formula (1), which shows strong emission, is useful as a light-emitting material contained in the light-emitting layer of organic EL elements. In addition, the compound is also useful as the light-emitting layer host material or the electron-injecting/transporting material in organic EL elements. It can also be used as a material for fluorescent indicators for detection of trace elements, with its favorable emission characteristics.

### BRIEF DESCRIPTION OF THE DRAWINGS

Fig. 1 is the ¹H-NMR spectrum of compound 16.
Fig. 2 is the MS spectrum of compound 16.
Fig. 3 is the MS spectrum of compound 20.
Fig. 4 is the ¹H-NMR spectrum of compound 47.
Fig. 5 is the MS spectrum of compound 47.

### BEST MODE OF CARRYING OUT THE INVENTION

Hereinafter, the compound represented by General Formula (1) according to the present invention will be described in detail. In General Formula (1) above, R¹ and R² each independently represent a substituted or unsubstituted alkyl group, a substituted or unsubstituted aryl group, or a substituted or unsubstituted heterocyclic group.

Examples of the alkyl groups include, but are not limited to, methyl, ethyl, propyl, butyl, sec-butyl, tert-butyl, pentyl, hexyl, heptyl, octyl, and stearyl groups. The number of carbons in the alkyl group is preferably 1 to 18, more preferably 1 to 8.

Examples of the aryl groups include, but are not limited to, phenyl, naphthyl, biphenyl, anthranyl, phenanthryl, fluorenyl, pyrenyl, and perylenyl groups.

Examples of the heterocyclic groups include, but are not limited to, pyridinyl, pyrazinyl, pyrimidinyl, pyridazinyl, triazinyl, indolynyl, quinolinyl, acridinyl, pyrrolidinyl, dioxanyl, piperidinyl, morpholinyl, piperazinyl, carbazolyl, furanyl, thiophenyl, oxazolyl, oxadiazolyl, benzoxazolyl, thiazolyl, thiadiazolylp, benzothiazolyl, triazolyl, imidazolyl, benzimidazolyl, bipyridyl, phenanthrolinyl, quinolyl, and acrydinyl groups.

Favorable among the groups above are substituted or unsubstituted aryl groups having rings of 6 to 30 carbon atoms such as phenyl, naphthyl, biphenyl, anthranyl, phenanthryl and perylenyl groups and substituted or unsubstituted heterocyclic groups having rings of 3 to 30 carbon atoms and 1 to 10 heteroatoms such as pyridyl, quinolinyl, carbazolyl, furanyl, thiophenyl, oxazolyl, bipyridyl and phenanthrolinyl groups. More preferable are phenyl, naphthyl, anthranyl, pyridyl, quinolinyl and oxazolyl groups.

Each of the alkyl, aryl and heterocyclic groups may be substituted or may not be substituted. Examples of the substituent groups include a hydroxyl group, a carboxylic acid group, halogen atoms, a cyano group, a nitro group, substituted or unsubstituted acyl groups, substituted or unsubstituted alkyloxycarbonyl groups, substituted or unsubstituted aryloxycarbonyl groups, substituted or unsubstituted acyloxy groups, substituted or unsubstituted acylamino groups, substituted or unsubstituted N-alkylcarbamoyl groups, substituted or unsubstituted N-arylcarbamoyl groups, substituted or unsubstituted alkyl groups, substituted or unsubstituted aryl groups, substituted or unsubstituted alkyloxy groups, substituted or unsubstituted aryloxy groups, substituted or unsubstituted alkylthio groups, substituted or unsubstituted arylthio groups, substituted or unsubstituted amino groups, substituted or unsubstituted heterocyclic groups and the like.

Examples of the halogen atoms include, but are not limited to, chlorine, bromine, iodine, and fluorine. Among the halogens above, fluorine and chlorine are referable.

Examples of the acyl groups include, but are not limited to, formyl, acetyl, propionyl, butyryl, isobutyryl, cyclohexylcarbonyl, benzoyl, and phenylacetyl groups. Among them, acetyl, propionyl and phenylacetyl groups are preferable.

Examples of the alkyloxycarbonyl groups include, but are not limited to, methyloxycarbonyl, ethyloxycarbonyl, propyloxycarbonyl, isopropyloxycarbonyl, cyclohexyloxycarbonyl, and benzyloxycarbonyl groups. Among them, methyloxycarbonyl, ethyloxycarbonyl and cyclohexyloxycarbonyl groups are preferable.

Examples of the aryloxycarbonyl groups include, but are not limited to, phenyloxycarbonyl, tolyloxycarbonyl, biphenyloxycarbonyl, naphthyloxycarbonyl, anthryloxycarbonyl, pyrenyloxycarbonyl, and perylenyloxycarbonyl groups. Among them, phenyloxycarbonyl, tolyloxycarbonyl and biphenyloxycarbonyl groups are preferable.

Examples of the acyloxy group include, but are not limited to, formyloxy, acetyloxy, propionyloxy, butyryloxy, isobutyryloxy, cyclohexylcarbonyloxy, benzoyloxy, and phenylacetyloxy groups. Among them, acetyloxy, cyclohexylcarbonyloxy and phenylacetyloxy groups are preferable.

Examples of the acylamino group include, but are not limited to, formylamino, acetylamino, propionylamino, butyrylamino, isobutyrylamino, and benzoylamino groups. Among them, acetylamino, propionylamino and butylamino groups are preferable.

Examples of the N-alkylcarbamoyl groups include, but are not limited to, carbamoyl, N-methylcarbamoyl, N-ethylcarbamoyl, N-propylcarbamoyl, N-isopropylcarbamoyl, N-cyclohexylcarbamoyl, and N-benzylcarbamoyl groups. Among them, N-methylcarbamoyl, N-ethylcarbamoyl and N-cyclohexylcarbamoyl groups are preferable.

Examples of the N-arylcarbamoyl groups include, but are not limited to, N-phenylcarbamoyl, N-tolylcarbamoyl, N-biphenylylcarbamoyl, N-naphthylcarbamoyl, N-anthrylcarbamoyl, N-pyrenylcarbamoyl, and N-perylenylcarbamoyl groups. Among them, N-phenylcarbamoyl, N-tolylcarbamoyl and N-biphenylylcarbamoyl groups are preferable.

Examples of the alkyl groups include, but are not limited to, methyl, ethyl, propyl, butyl, sec-butyl, tert-butyl, pentyl, hexyl, heptyl, octyl, stearyl, and trichloromethyl groups. Among them, methyl, ethyl, propyl, butyl, sec-butyl and tert-butyl groups are preferable.

Examples fo the aryl groups include those described for R¹ and R² above.

Examples of the alkyloxy groups include, but are not limited to, methoxy, n-butoxy, tert-butoxy, trichloromethoxy, trifluoroethoxy, pentafluoropropoxy, 2,2,3,3-tetrafluoropropoxy, 1,1,1,3,3,3-hexafluoro-2-propoxy, 6-(perfluoroethyl)hexyloxy, and benzyloxy groups. Among them, methoxy, n-butoxy and tert-butoxy groups are preferable.

Examples of the aryloxy groups include, but are not limited to, phenoxy, p-nitrophenoxy, p-tert-butylphenoxy, 3-fluorophenoxy, pentafluorophenyl, 3-trifluoromethylphenoxy, naphthyloxy, and anthryloxy groups. Among them, phenoxy, naphthyloxy and anthryloxy groups are preferable.

Examples of the alkylthio group include, but are not limited to, methylthio, ethylthio, tert-butylthio, hexylthio, octylthio, and trifluoromethylthio groups. Among them, methylthio, ethylthio and tert-butylthio groups are preferable.

Examples of the arylthio groups include, but are not limited to, phenylthio, p-nitrophenylthio, p-tert-butylphenylthio, 3-fluorophenylthio, pentafluorophenylthio, and 3-trifluoromethylphenylthio groups. Among them, phenylthio and p-tert-butylphenylthio groups are preferable.

Examples of the amino groups include, but are not limited to, amino, methylamino, diethylamino, ethylamino, diethylamino, dipropylamino, dibutylamino, and diphenylamino groups. Among them, diethylamino, dipropylamino, dibutylamino and diphenylamino groups are preferable.

Examples of the heterocyclic groups include those described for R¹ and R² above.

Hereinafter, typical examples of the compounds represented by General Formula (1) according to the present invention (exemplary compounds 1 to 104) for use in organic EL elements are listed in Table 1, but the present invention is not restricted by these compounds.

**[Table 1]**

| | | | |
|---|---|---|---|
| | | | |
| **1** | 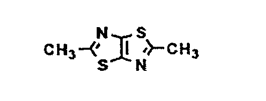 | **2** | 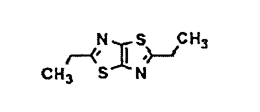 |
| **3** | 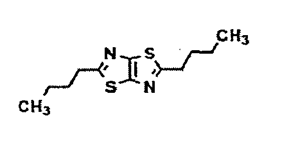 | **4** | 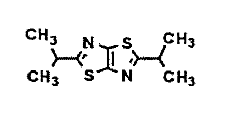 |
| **5** | 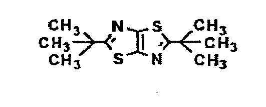 | **6** | 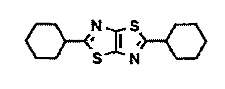 |
| **7** | 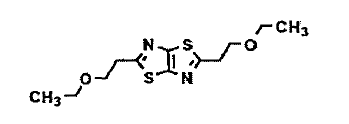 | **8** | 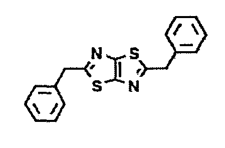 |
| **9** | 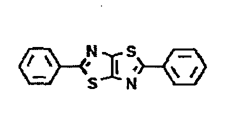 | **10** | 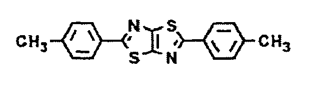 |
| **11** | 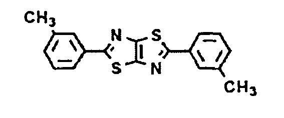 | **12** | 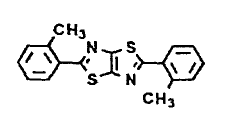 |
| **13** | 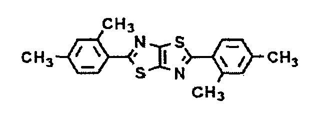 | **14** | 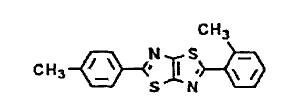 |
| **15** | 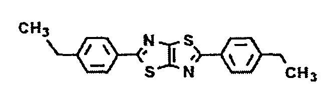 | **16** | 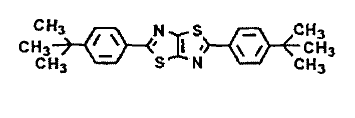 |
| **17** | 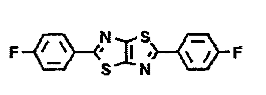 | **18** | 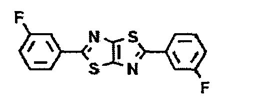 |
| **19** | 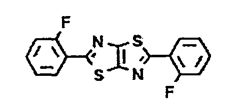 | **20** | 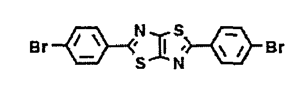 |
| **21** | 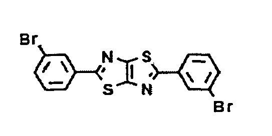 | **22** | 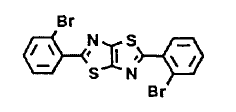 |
| **23** | 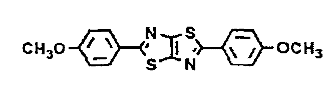 | **24** | 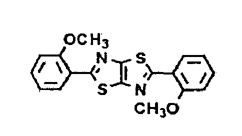 |
| **25** | 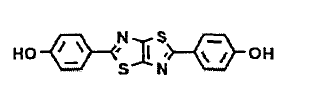 | **26** | 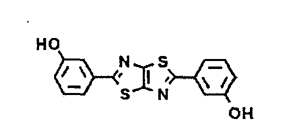 |
| **27** | 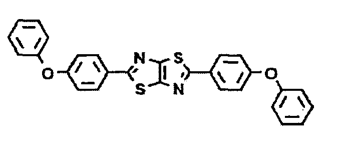 | **28** | 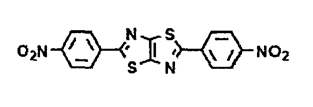 |
| **29** | 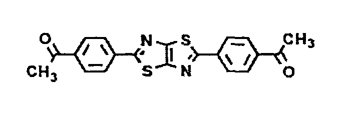 | **30** | 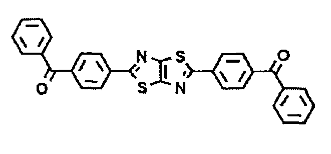 |
| **31** | 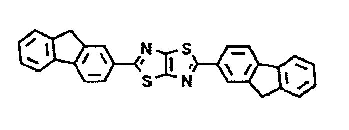 | **32** | 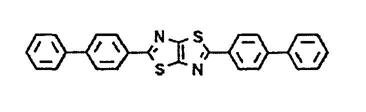 |
| **33** | 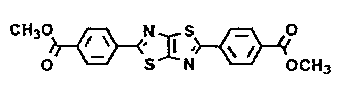 | **34** | 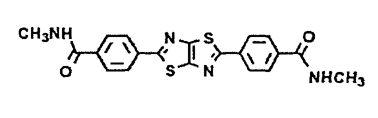 |
| **35** | 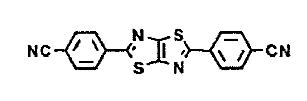 | **36** | 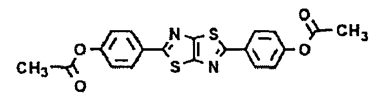 |
| **37** | 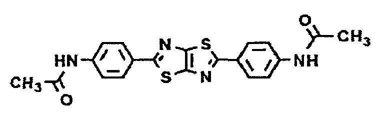 | **38** | 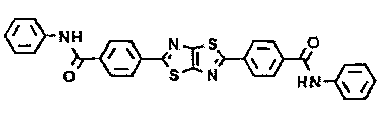 |
| **39** | 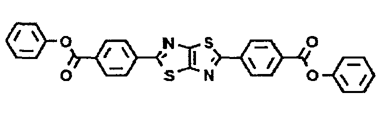 | **40** | 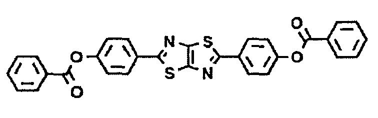 |
| **41** | 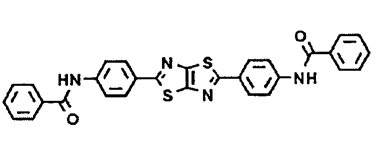 | **42** | 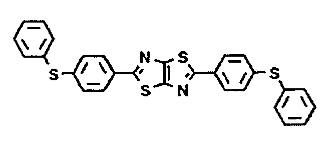 |
| **43** | 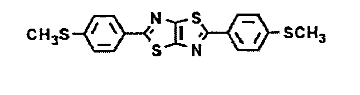 | **44** | 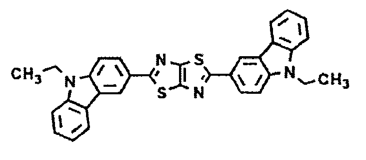 |
| **45** | 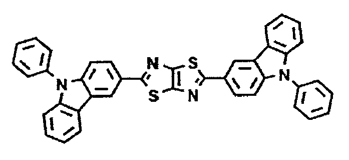 | **46** | 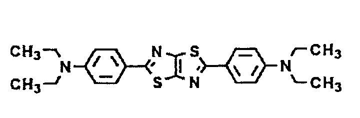 |
| **47** | 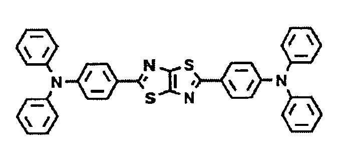 | **48** | 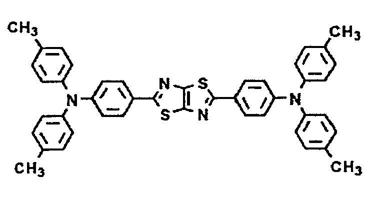 |
| **49** | 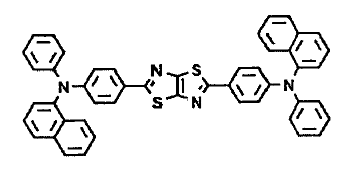 | **50** | 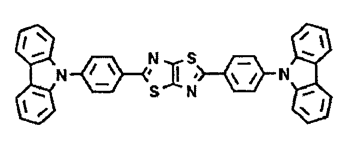 |
| **51** | 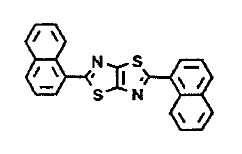 | **52** | 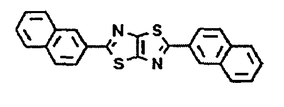 |
| **53** | 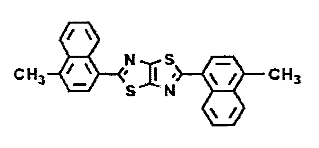 | **54** | 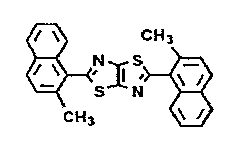 |
| **55** | 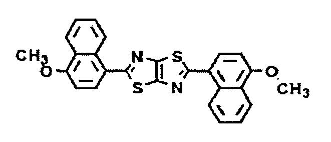 | **56** | 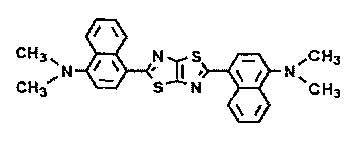 |
| **57** | 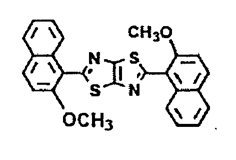 | **58** | 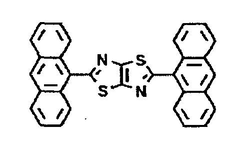 |
| **59** | 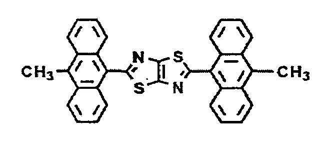 | **60** | 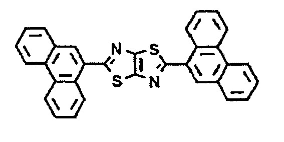 |
| **61** | 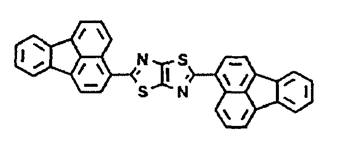 | **62** | 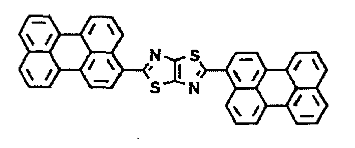 |
| **63** | 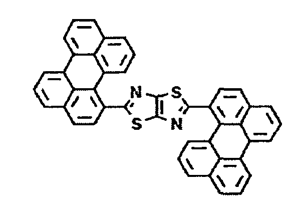 | **64** | 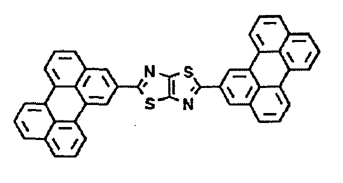 |
| **65** | 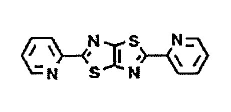 | **66** | 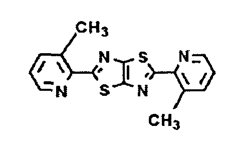 |
| **67** | 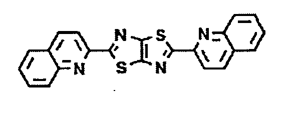 | **68** | 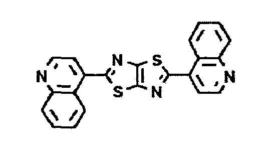 |
| **69** | 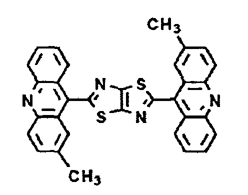 | **70** | 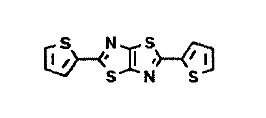 |
| **71** | 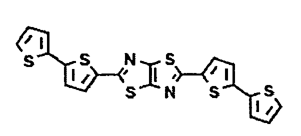 | **72** | 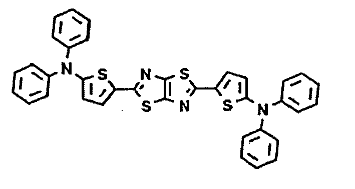 |
| **73** | 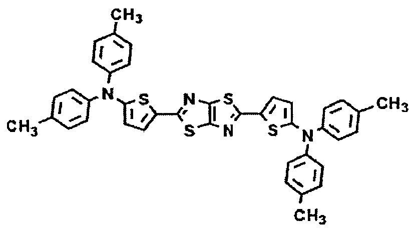 | **74** | 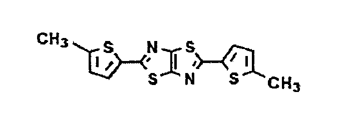 |
| **75** | 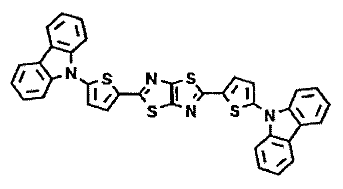 | **76** | 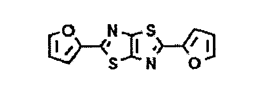 |
| **77** | 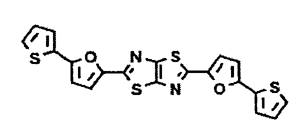 | **78** | 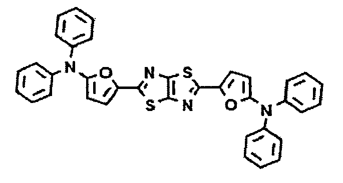 |
| **79** | 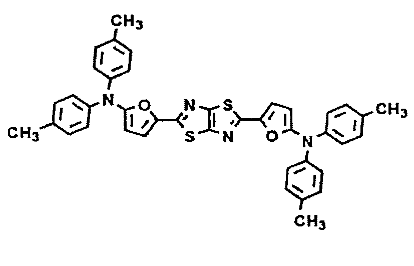 | **80** | 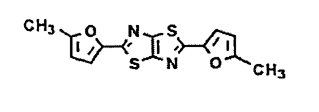 |
| **81** | 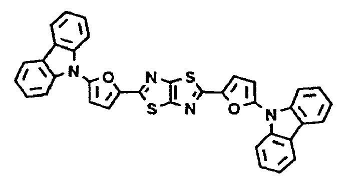 | **82** | 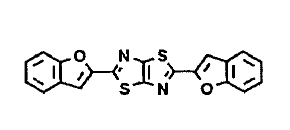 |
| **83** | 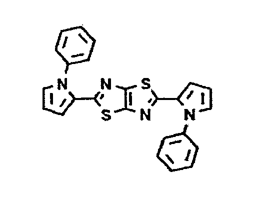 | **84** | 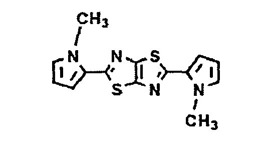 |
| **85** | 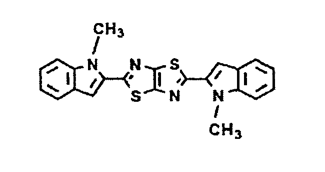 | **86** | 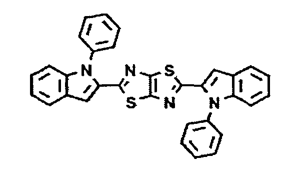 |
| **87** | 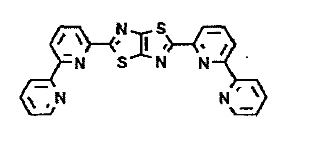 | **88** | 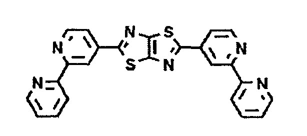 |
| **89** | 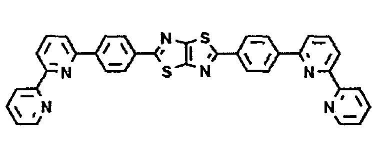 | **90** | 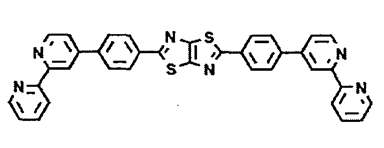 |
| **91** | 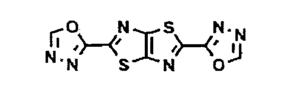 | **92** | 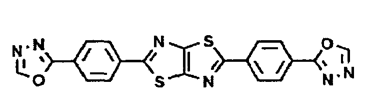 |
| **93** | 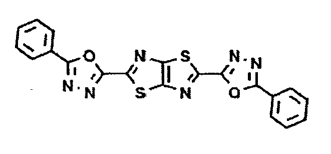 | **94** | 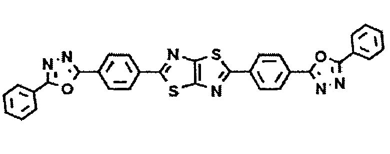 |
| **95** | 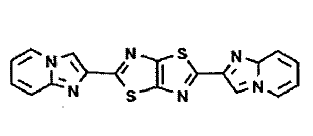 | **96** | 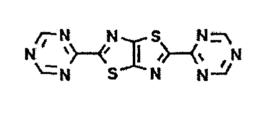 |
| **97** | 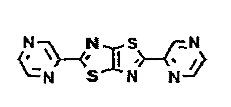 | **98** | 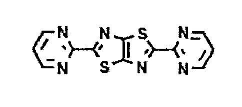 |
| **99** | 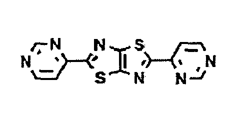 | **100** | 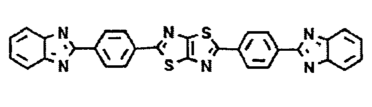 |
| **101** | 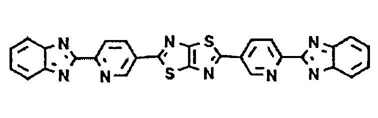 | **102** | 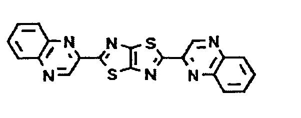 |
| **103** | 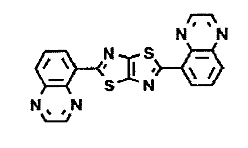 | **104** | 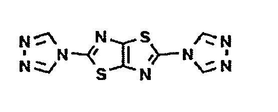 |

The compounds represented by General Formula (1) can be produced by known methods, and, for example, J. Amer. Chem. Soc., 82, p. 2719 to 2724, 1960 disclosed that the compound was obtained by stirring rubeanic acid and an aldehyde under heat in a high-boiling-point solvent such as phenol. The disclosure of the document is incorporated herein by reference.

The compound represented by General Formula (1) shows strong light emission and thus, can be used as a raw material for fluorescent pigments and fluorescence paints, fluorescence indicators, electronics materials and the like. In particular, a long-life organic EL element superior in color purity and brightness can be obtained by using the compound as a raw material for organic EL element.

The organic EL element is composed of an element comprising an anode, a cathode, and a single-layered or multilayered organic layer formed between them. The single-layered organic EL element is an element having an anode, a cathode and a light-emitting layer formed between them. The multilayered organic EL element is an element additionally having a hole-injecting layer, a hole-transporting layer, a hole-blocking layer, an electron-injecting layer, an electron-transporting layer or the like for facilitating injection of positive hole or electron into the light-emitting layer or for facilitating recombination between positive hole and electron in the light-emitting layer. Examples of the element configurations of multilayered organic EL element include:
(1) anode/hole-injecting layer/light-emitting layer/cathode,
(2) anode/hole-injecting layer/hole-transporting layer/light-emitting layer/cathode,
(3) anode/hole-injecting layer/light-emitting layer/electron-injecting layer/cathode,
(4) anode/hole-injecting layer/hole-transporting layer/light-emitting layer/electron-injecting layer/cathode,
(5) anode/hole-injecting layer/light-emitting layer/hole-blocking layer/electron-injecting layer/cathode,
(6) anode/hole-injecting layer/hole-transporting layer/light-emitting layer/hole-blocking layer/electron-injecting layer/cathode,
(7) anode/light-emitting layer/hole-blocking layer/electron-injecting layer/cathode,
(8) anode/light-emitting layer/electron-injecting layer/cathode,
(9) anode/hole-injecting layer/hole-transporting layer/light-emitting layer/electron-transporting layer/electron-injecting layer/cathode, and
(10) anode/hole-injecting layer/light-emitting layer/hole-blocking layer/electron-transporting layer/electron-injecting layer/cathode.

The light-emitting layer may comprise the compound according to the present invention. If needed, it may comprise, in addition to the compound according to the present invention, a known light-emitting material, a doping material, a hole-injecting material or an electron-injecting material. A luminescent brightness and a luminous efficiency can be improved depending on the material used and the composition thereof and emits light in a variety of colors such as red, blue and green. In addition, the light-emitting layer, if it contains multiple light-emitting materials, can show white emission.

Examples of the light-emitting or doping materials for use in the light-emitting layer together with the compound according to the present invention include, but are not limited to, anthracene derivatives, naphthalene derivatives, phenanthrene derivatives, pyrene derivatives, tetracene derivatives, coronene derivatives, chrysene derivatives, fluorescein derivatives, perylene derivatives, phthaloperylene derivatives, naphthaloperylene derivatives, perynone derivatives, phthaloperynone derivatives, naphthaloperynone derivatives, diphenylbutadiene derivatives, tetraphenylbutadiene derivatives, coumarin derivatives, oxadiazole derivatives, aldazine derivatives, bisbenzoxazoline derivatives, bisstyryl derivatives, diketopyrrolopyrrole derivatives, pyrazine derivatives, cyclopentadiene derivatives, quinoline metallocomplex derivatives, diphenylethylene derivatives, vinylanthracene derivatives, carbazole derivatives, pyran derivatives, thiopyran derivatives, polymethine derivatives, merocyanine derivatives, imidazole-chelated oxynoid compounds, quinacridone derivatives, rubrene derivatives, and fluorescent dyes for dye laser and bleaching.

Among them, naphthalene derivatives, anthracene derivatives, phenanthrene derivatives, pyrene derivatives, tetracene derivatives, perylene derivatives, carbazole derivatives, diketopyrrolopyrrole derivatives and quinoline metal complexes are favorable. These derivatives additionally substituted with diarylamino groups are more preferable.

In addition, the light-emitting layer may comprise one or more polymers such as an insulating resin such as polystyrene, polycarbonate, polyacrylate, polyester, polyamide, polyurethane, polysulfone, polymethyl methacrylate, polymethyl acrylate, or cellulose or the copolymer thereof, a photoconductive resin such as poly-N-vinylcarbazole or polysilane, and a conductive resin such as polythiophene or polypyrrole. The light-emitting layer may contain an antioxidant, an ultraviolet absorbent, a plasticizer or the like for improvement in film-forming property and prevention of pinholes in the layer.

The contents of the compound according to the present invention and the other compounds used in combination in the light-emitting layer are not particular limited, and thus, any compound may become the principal component. More specifically, the compound according to the present invention may be the main material (host material) or the doping material in the light-emitting layer.

A hole-injecting material giving a hole-injecting layer having a favorable hole-injecting efficiency to the light-emitting layer and being superior in adhesiveness at the interface to the anode and in thin film-forming efficiency is used as the hole-injecting layer. Alternatively, a hole-transporting material higher in hole-transporting efficiency of transporting holes smoothly into the light-emitting layer is used as the hole-transporting layer. Examples of the hole-injecting or hole-transporting material include, but are not limited to, low-molecular-weight compounds such as phthalocyanine derivatives, naphthalocyanine derivatives, porphyrin derivatives, oxadiazole derivatives, triazole derivatives, imidazole derivatives, imidazolone derivatives, imidazolethione derivatives, pyrazoline derivatives, pyrazolone derivatives, tetrahydroimidazole derivatives, oxazole derivatives, oxadiazole derivatives, hydrazone derivatives, acyl hydrazone derivatives, stilbene derivatives, and aromatic tertiary amine derivative; and polymer compounds such as polyvinyl carbazole derivatives and polysilane derivative.

Among them, the hole-injecting or hole-transporting material is preferably an aromatic tertiary amine derivative or a phthalocyanine derivative. Examples of the aromatic tertiary amine derivatives include N,N'-diphenyl-N,N'-(3-methylphenyl)-1,1'-biphenyl-4,4'-diamine, N,N,N',N'-(4-methylphenyl)-1,1'-phenyl-4,4'-diamine, N,N,N',N'-(4-methylphenyl)-1,1'-biphenyl-4,4'-diamine, N,N'-diphenyl-N,N'-dinaphthyl-1,1'-biphenyl-4,4'-diamine, N,N'-(methylphenyl)-N,N'-(4-n-butylphenyl)-phenanthrene-9,10-diamine, N,N-bis(4-di-4-tolylaminophenyl)-4-phenyl-cyclohexane, and oligomers or polymers having such an aromatic tertiary amine skeleton. Examples of the phthalocyanine (hereinafter, referred to as Pc) derivatives include phthalocyanine derivative such as H₂Pc, CuPc, CoPc, NiPc, ZnPc, PdPc, FePc, MnPc, ClAlPc, ClGaPc, ClInPc, ClSnPc, Cl₂SiPc, (HO)AlPc, (HO)GaPc, VOPc, TiOPc, MoOPc, and GaPc-O-GaPc, and these compounds are used particularly favorably as a hole-injecting material.

An electron-injecting material capable of forming an electron-injecting layer that has excellent electron-injecting efficiency to the light-emitting layer and is superior in adhesiveness to the cathode surface and thin film-forming efficiency is used for preparation of the electron-injecting layer. Alternatively, an electron-transporting material transporting electrons smoothly to the light-emitting layer is used as the electron-transporting layer. Examples of the electron-injecting materials include, in addition to the compound according to the present invention, metallocomplex compounds, nitrogen-containing five-membered ring derivatives, fluorenone derivatives, anthraquinodimethane derivatives, diphenoquinone derivatives, thiopyranedioxide derivatives, perylenetetracarboxylic acid derivatives, fluorenylidenemethane derivatives, anthrone derivatives, silole derivatives, calcium acetylacetonate,and sodium acetate. Other examples include inorganic/organic composite materials of bathophenanthroline doped with a metal such as cesium (Preprint of Annual Meeting of Soc. Polymer Science, Japan, 50, 4, p.660, 2001) and BCP, TPP, T5MPyTZ and others described in Preprint of 50th Meeting, JSAP and Related Societies No.3, p.1402, 2003. However, the material is not particularly limited, if it forms a thin film desirable in production of the element, receives electrons from the cathode, and transports the electrons. The disclosures of the documents above are incorporated by reference herein. Examples of electron-transporting material include, but are not limited to, in addition to the compound according to the present invention, metallocomplex compounds, nitrogen-containing five-membered ring derivatives, fluorenone derivatives, anthraquinodimethane derivatives, diphenoquinone derivatives, thiopyranedioxide derivatives, perylenetetracarboxylic acid derivatives, fluorenylidenemethane derivatives, anthrone derivatives, silole derivatives, calcium acetylacetonate, and sodium acetate.

Among the electron-injecting materials and the electron-transporting materials above, particularly effective are the compounds according to the present invention, metallocomplex compounds and nitrogen-containing five-membered ring derivatives. Examples of the favorable metallocomplex compounds include aluminum complex compounds such as tris(8-quinolinolato)aluminum, tris(2-methyl-8-quinolinolato)aluminum, tris(5-phenyl-8-quinolinolato)aluminum, bis(8-quinolinolato)(1-naphtholato)aluminum, bis(8-quinolinolato)(2-naphtholato)aluminum, bis(8-quinolinolato)(phenolato)aluminum, bis(8-quinolinolato)(4-cyano-1-naphtholato)aluminum, bis(4-methyl-8-quinolinolato)(1-naphtholato)aluminum, bis(5-methyl-8-quinolinolato)(2-naphtholato)aluminum, bis(5-phenyl-8-quinolinolato)(phenolato)aluminum, bis(5-cyano-8-quinolinolato)(4-cyano-1-naphtholato)aluminum, bis(8-quinolinolato)chloroaluminum, and bis(8-quinolinolato)(o-cresolato)aluminum; gallium complex compounds such as tris(8-quinolinolato)gallium, tris(2-methyl-8-quinolinolato)gallium, tris(2-methyl-5-phenyl-8-quinolinolato)gallium, bis(2-methyl-8-quinolinolato)(1-naphtholato)gallium, bis(2-methyl-8-quinolinolato)(2-naphtholato)gallium, bis(2-methyl-8-quinolinolato)(phenolato)gallium, bis(2-methyl-8-quinolinolato)(4-cyano-1-naphtholato)gallium, bis(2,4-dimethyl-8-quinolinolato)(1-naphtholato)gallium, bis(2,5-dimethyl-8-quinolinolato)(2-naphtholato)gallium, bis(2-methyl-5-phenyl-8-quinolinolato)(phenolato)gallium, bis(2-methyl-5-cyano-8-quinolinolato)(4-cyano-1-naphtholato)gallium, bis(2-methyl-8-quinolinolato) chlorogallium, and bis(2-methyl-8-quinolinolato)(o-cresolato)gallium; and metallocomplex compounds such as 8-quinolinolatolithium, bis(8-quinolinolato)copper, bis(8-quinolinolato)manganese, bis(10-hydroxybenzo[h]quinolinato)beryllium, bis(8-quinolinolato)zinc, and bis(10-hydroxybenzo[h]quinolinato)zinc.

Examples of the favorable nitrogen-containing five-membered ring derivative include oxazole derivatives, thiazole derivatives, oxadiazole derivatives, thiadiazole derivatives and triazole derivative; and specifically, 2,5-bis(1-phenyl)-1,3,4-oxazole, 2,5-bis(1-phenyl)-1,3,4-thiazole, 2,5-bis(1-phenyl)-1,3,4-oxadiazole, 2-(4',-tert-butylphenyl)-5-(4"-biphenyl)1,3,4-oxadiazole, 2,5-bis(1-naphthyl)-1,3,4-oxadiazole, 1,4-bis[2-(5-phenyloxadiazolyl)]benzene, 1,4-bis[2-(5-phenyloxadiazolyl)-4-tert-butylbenzene], 2-(4',-tert-butylphenyl)-5-(4"-biphenyl)-1,3,4-thiadiazole, 2,5-bis(1-naphthyl)-1,3,4-thiadiazole, 1,4-bis[2-(5-phenylthiadiazolyl)]benzene, 2-(4',-tert-butylphenyl)-5-(4"-biphenyl)-1,3,4-triazole, 2,5-bis(1-naphthyl)-1,3,4-triazole, and 1,4-bis[2-(5-phenyltriazolyl)]benzene.

A hole-blocking material blocking positive holes from entering into the electron-injecting layer through the light-emitting layer that is superior in thin film-forming efficiency is used for the hole-blocking layer. Examples of the hole-blocking materials include, but are not limited to, aluminum complex compounds such as bis(8-quinolinolato)(4-phenylphenolato)aluminum, gallium complex compounds such as bis(2-methyl-8-quinolinolato)(4-phenylphenolato)gallium, and nitrogen-containing condensed aromatic compounds such as 2,9-dimethyl-4,7-diphenyl-1,10-phenanthroline (BCP).

The light-emitting layer in the organic EL element may contain, as needed in addition to the material for organic EL elements according to the present invention, another light-emitting material and a doping material as well as the hole-injecting material and the electron-injecting material described above in combination of two or more. In addition, each of the hole-injecting, light-emitting and electron-injecting layers may have a two or more-layered structure.

Examples of the materials used as the anode of the organic EL element according to the present invention include, but are not limited to, carbon, metals such as aluminum, vanadium, iron, cobalt, nickel, tungsten, silver, gold, platinum, and palladium and the alloys thereof; conductive metal oxides such as zinc oxide, tin oxide, indium oxide and indium tin oxide (ITO); conductive polymers such as polythiophene, polypyrrole and polyaniline. In particular, the conductive material used as the anode of the organic EL element according to the present invention is preferably lower in resistance, and thus, ITO glass or NESA glass is used favorably.

The material used as the cathode of the organic EL element according to the present invention is not particularly limited, if it is a material allowing efficient injection of electrons into the organic EL element, and typical examples thereof include platinum, gold, silver, copper, iron, tin, zinc, aluminum, indium, chromium, lithium, sodium, potassium, calcium, and magnesium, and the alloys thereof. The alloy is preferably an alloy containing a low-work-function metal such as lithium, sodium, potassium, calcium, or magnesium. Examples thereof include, but are not limited to, magnesium/silver, magnesium/indium, and lithium/aluminum. Alternatively, an inorganic salt such as lithium fluoride may be used, replacing the low-work function metal. Such a cathode can be prepared by a method known in the art such as resistance-heating, electron beam irradiation, sputtering, ion plating, or coating. The anode and the cathode described above may have a layered structure of two or more layers as needed.

For efficient emission of light from the organic EL element according to the present invention, the material for the light-emitting substrate is desirably transparent. Specifically, the light transmittance of the element in the emission wavelength range is preferably 50% or more, more preferably 90% or more. The material for the substrate is not particularly limited, if it has suitable mechanical and thermal strengths and is transparent. Examples of the materials include glass as well as transparent polymers such as polyethylene, polyether sulfone, polypropylene, and PET.

Each layer on the organic EL element according to the present invention can be formed by a dry or wet film-forming method. Examples of the dry film-forming methods include, but are not limited to, vacuum deposition, electron beam irradiation, sputtering, plasma, and ion plating. Examples of the wet film-forming methods include, but are not limited to, spin coating, dipping, and flow coating. The thickness of each layer is not particularly limited, but an excessively thick film demands large applied voltage for obtaining a particular emission intensity, leading to deterioration in efficiency, while an excessively thin film has a greater number of pin holes for example, making it difficult to obtain a sufficient luminescent brightness even when electric field is applied. Thus, the thickness of each layer is preferably in the range of 1 nm to 1 µm, more preferably 10 nm to 0.2 µm.

A protective layer may be formed on the surface of the organic EL element, or the entire element may be coated or sealed for example with a resin, for improvement in stability to temperature, humidity, atmosphere and others.
In particular, when the entire element is coated or sealed, a photocuring resin that hardens by photoirradiation is used favorably.

As described above, the organic EL element can emit light superior in color purity and brightness at low operating voltage. Therefore, the organic EL element can be used in various applications including flat panel displays of wall-hung television, flat panel illuminators, light sources for copying machines, and printers, light sources for liquid crystal displays, measuring meters, display plates, marker lights and others.

### EXAMPLES

Hereinafter, the present invention will be described more specifically with reference to Examples, but it should be understood that the present invention is not restricted by the following Examples. The blending ratio in Examples below is a ratio by weight, unless specified otherwise.

### Preparative Example 1

### (Preparation of 2,5-bis(4-t-butylphenyl)thiazolo[4,5-d]thiazole <exemplary compound 16>)

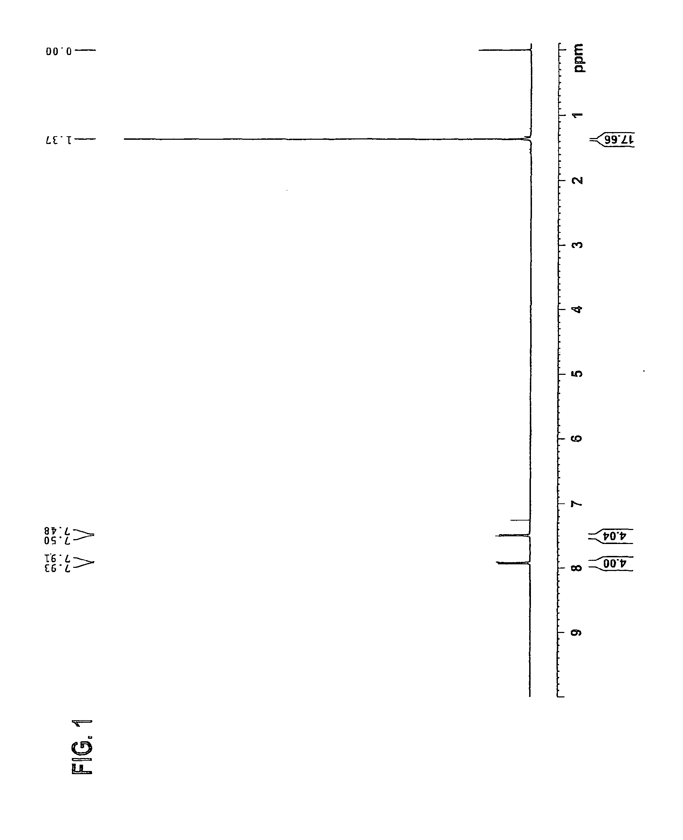

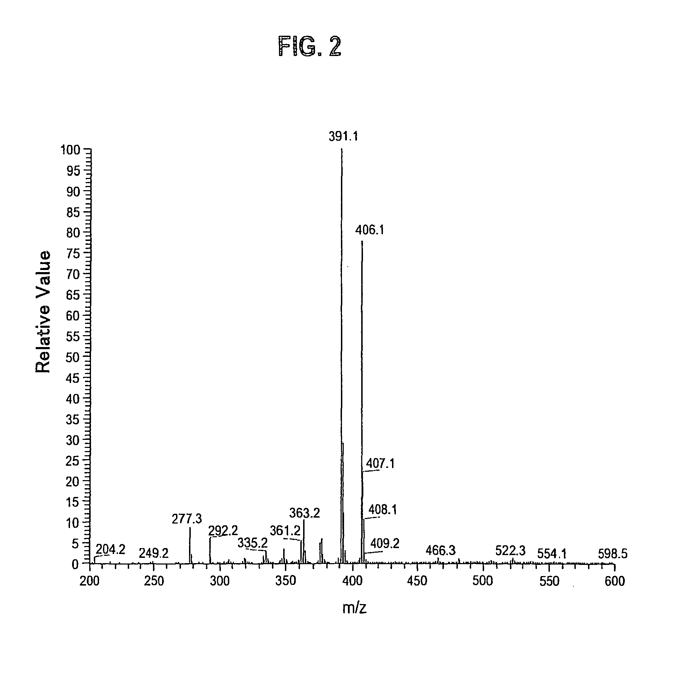

Rubeanic acid (5.00 g), 4-t-butylbenzaldehyde (23.62 g) and phenol (20.0 g) were placed in a reaction container, and the mixture was heated under reflux under a nitrogen environment for 5 hours. The reaction solution was cooled to room temperature; methanol (100 mL) was added; and the resulting pale yellow precipitate was collected by filtration, to give compound 16 (8.80 g, 52%). The ¹H-NMR spectrum (AVANCE 400, manufactured by Bruker Biospin) and the MS spectrum (Polaris Q, manufactured by Thermo Electron K.K.) of the compound 16 obtained are shown in Figs. 1 and 2. The compound 16 was identified, based on the following analytical results.
¹H-NMR (400 MHz, CDCl₃): 7.92 (4H, d, J=8.6 Hz), 7.49 (4H, d, J=8.6 Hz), 1.37 (18H, s) (Fig. 1). m/z (EI⁺): 406 (M⁺) (Fig. 2).

### Preparative Example 2

### (Preparation of 2,5-bis(4-bromophenyl)thiazolo[4,5-d]thiazole <exemplary compound 20>)

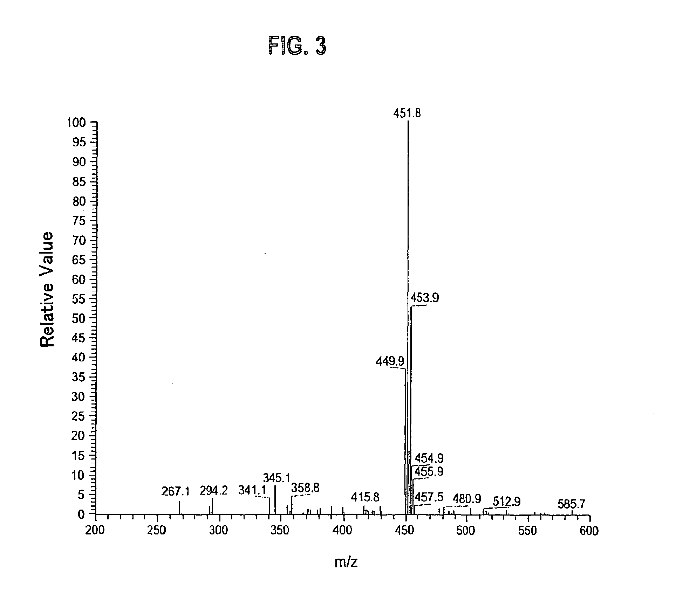

Rubeanic acid (5.00 g), p-bromobenzaldehyde (25.00 g) and phenol (20.0 g) were placed in a reaction container, and the mixture was heated under reflux under a nitrogen environment for 1 hour. The reaction solution was cooled to room temperature; ethyl acetate (100 mL) was added; and the resulting yellowish brown precipitate was collected by filtration to give compound 20 (7.29 g, 39%). The MS spectrum (Polaris Q, manufactured by Thermo Electron K.K.) of the compound 20 obtained is shown in Fig. 3. The compound 20 was identified, based on the following analytical results.
m/z (EI⁺): 450, 452, 454 (M⁺) (Fig. 3).

### Preparative Example 3

### (Preparation of 2,5-bis(4-diphenylaminophenyl)thiazolo[4,5-d]thiazole <exemplary compound 47>)

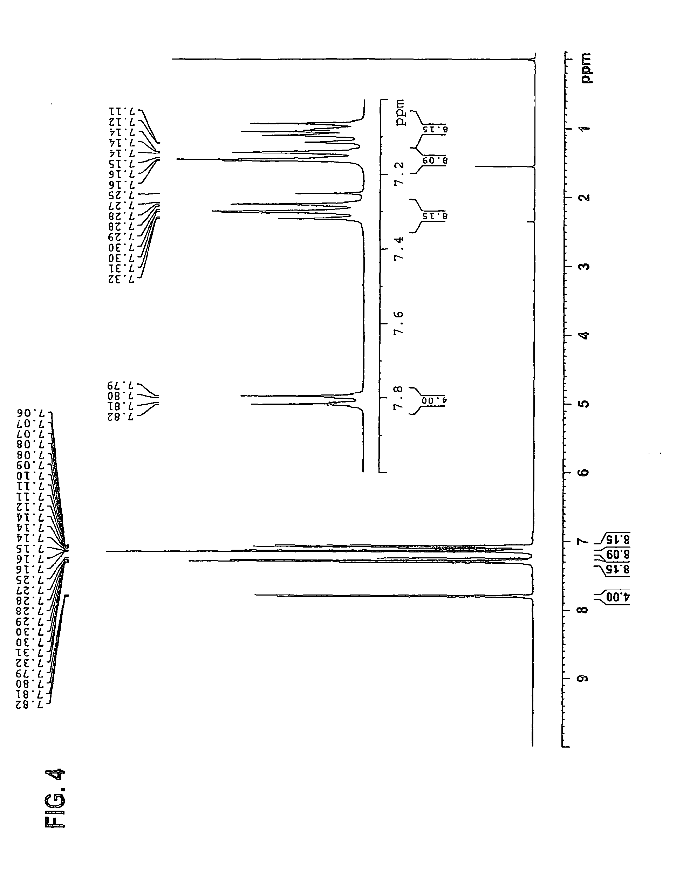

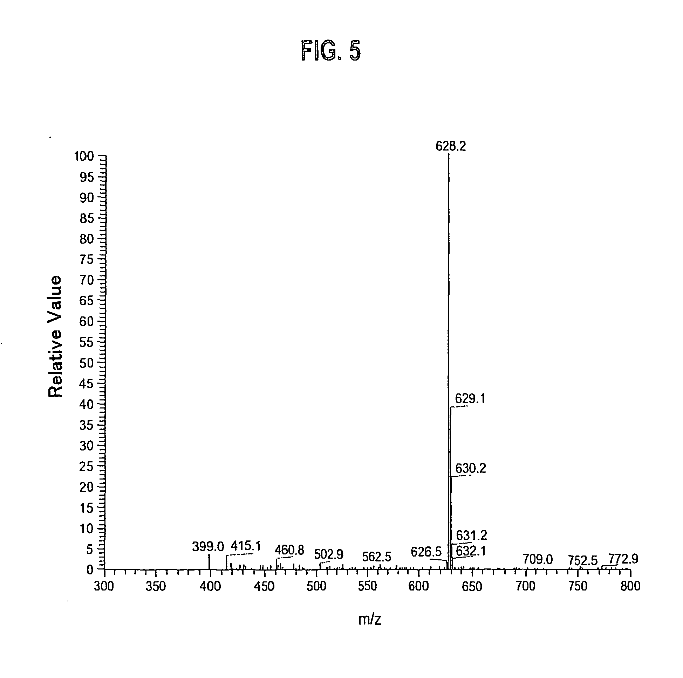

The compound 20 (3.5 g), diphenylamine (3.27 g), palladium acetate (0.174 g), toluene (30 mL), tri-t-butylphosphine (0.157 g) and sodium t-butoxide (1.86 g) were placed in a reaction container, and the mixture was heated at 100°C under a nitrogen environment for 2 hours. The reaction solution was cooled to room temperature; poured into methanol (150 mL); and the resulting brown precipitate was collected by filtration. The precipitate was purified by column chromatography (silica gel, chloroform) and recrystallized from toluene, to give compound 47 (3.88 g, 80%). The ¹H-NMR (AVANCE 400, manufactured by Bruker Biospin) and the MS spectrum (Polaris Q, manufactured by Thermo Electron K.K.) of the compound 47 obtained are shown in Figs. 4 and 5. The compound 47 was identified, based on the following analytical results.
¹H-NMR (400 MHz, CDCl₃): 7.82-7.79 (4H, m), 7.32-7.27 (8H, m), 7.16-7.14 (8H, m), 7.12-7.06 (8H, m) (Fig. 4).
m/z (EI⁺): 628 (M⁺) (Fig. 5).

### Example 1

A glass substrate carrying a transparent ITO electrode (anode) having a thickness of 200 nm was ultrasonicated in a neutral detergent solution, in acetone, and then in ethanol. The substrate was dried by using nitrogen gas, purified with UV/ozone, and fixed to a substrate holder of a vapor deposition apparatus, and the vapor deposition chamber was evacuated to 5×10⁻⁶ Torr. First, copper phthalocyanine (the following compound (A)) was deposited on the transparent ITO electrode at a vapor deposition speed of 0.2 nm/sec to a thickness of 10 nm, to give a hole-injecting layer. Then, N,N'-di(naphthalen-1-yl)-N,N'-diphenyl-benzidine (the following compound (B)) was deposited at a vapor deposition speed of 0.2 nm/sec to a thickness of 80 nm, to give a hole-transporting layer. Then, tris(8-quinolinolato)aluminum (the following compound (C)) and the exemplary compound 47 were codeposited thereon from different vapor deposition sources at a vapor deposition speed 0.2 nm/sec to a thickness of 40 nm (vapor deposition rate: 100:2), to give a light-emitting layer. Then, compound (C) was vapor-deposited thereon at a vapor deposition speed of 0.2 nm/sec to a thickness of 50 nm, to give an electron-transporting layer. And additionally, magnesium and silver were codeposited at a vapor deposition speed of 0.2 nm/sec to a thickness of 200 nm (weight ratio: 10:1), to give a cathode, thus giving an organic EL element.
Vapor deposition was carried out consecutively while the vapor deposition chamber was kept under reduced pressure.

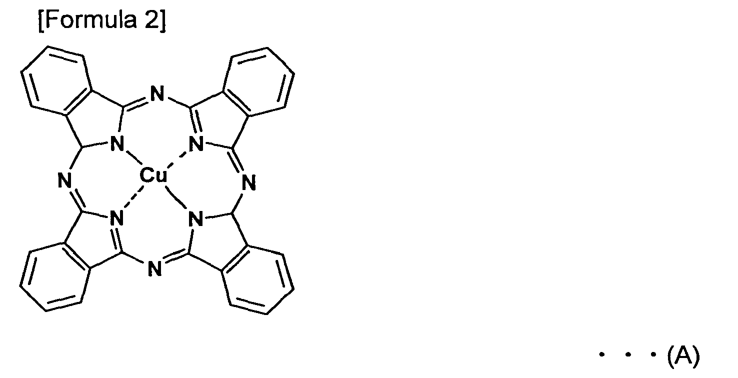

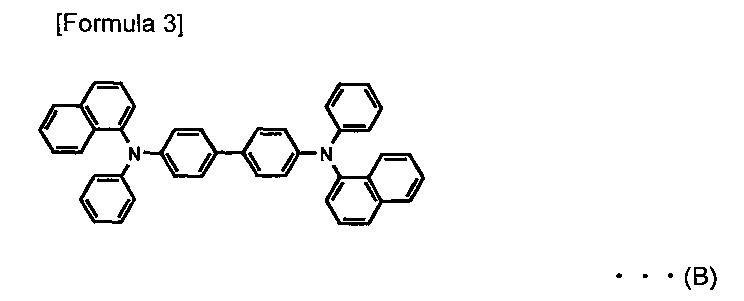

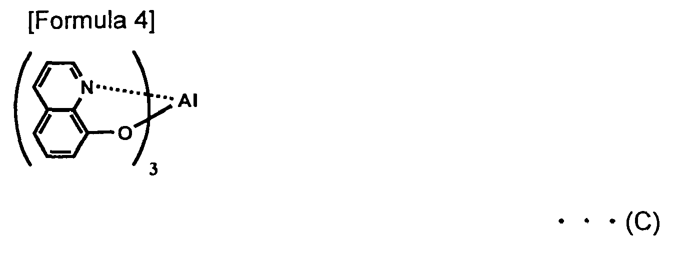

### Examples 2 to 5

An organic EL element was prepared in a similar manner to Example 1, except that the exemplary compound 47 was replaced with the compound shown in Table 2. Each element showed green to yellow emission, when a DC voltage was applied thereto under dry environment. The maximum brightness, the brightness at an operating voltage of 5 V, and the half lifetime under constant-current operation at an emission brightness of 1000 cd/m² of each element are shown in Table 2. The half lifetime is a relative value, with respect to 100 of the half life of the luminescence brightness of the organic EL element prepared in Example 1. The maximum brightness, the brightness and the half life were determined by using a color brightness meter CS-100 A (manufactured by Konica Minolta), as the element prepared was placed in an environment at room temperature under nitrogen stream.

### [Table 2]

**Table 2**

| Ex. | Compound | Max.brightness (cd/m²) | Brightness (cd/m²)*¹ | Half lifetime (relative value) | Emission color |
|---|---|---|---|---|---|
| 1 | 47 | 5600 | 140 | 100 | Yellow |
| 2 | 48 | 6900 | 110 | 9 8 | Yellow |
| 3 | 50 | 4800 | 190 | 110 | Yellow |
| 4 | 58 | 8300 | 210 | 108 | Green |
| 5 | 62 | 7300 | 150 | 101 | Orange |

| | | | | | |
|---|---|---|---|---|---|
| *1 : Brightness at 5V (cd/m²) | | | | | |

### Examples 6 to 10

An organic EL element was prepared in a similar manner to Example 1, except that the compound (C) used in the light-emitting layer was replaced with N,N'-di-(phenanthren-9-yl)-N,N'-diphenyl-benzidine (the following compound (D)) and the exemplary compound 47 with the compound shown in Table 3. Each element showed blue to green emission, when a DC voltage was applied thereto under dry environment. The maximum brightness, the brightness at an operating voltage of 5 V, and the half lifetime under constant-current operation at an emission brightness of 1000 cd/m² of each element are shown in Table 3. The half lifetime is a relative value, with respect to 100 of the half life of the luminescence brightness of the organic EL element prepared in Example 1.

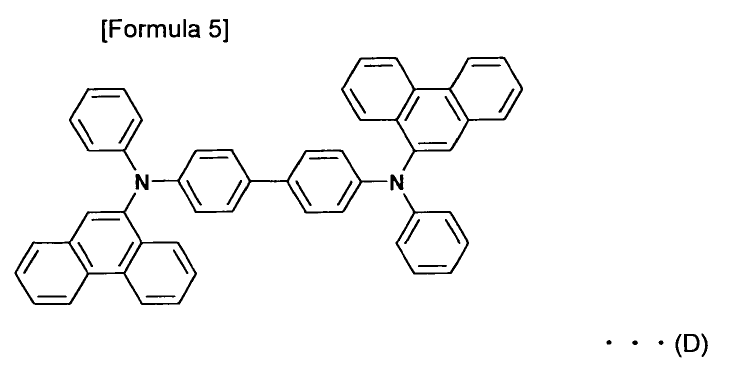

### [Table 3]

**Table 3**

| Ex. | Compound | Max. brightness (cd/m²) | Brightness (cd/m²)*¹ | Half lifetime (relative value) | Emission color |
|---|---|---|---|---|---|
| 6 | 16 | 6100 | 120 | 130 | Blue |
| 7 | 32 | 4200 | 130 | 153 | Bluish green |
| 8 | 51 | 4800 | 180 | 120 | Green |
| 9 | 70 | 7200 | 120 | 132 | Blue |
| 10 | 80 | 5800 | 200 | 128 | Bluish green |

| | | | | | |
|---|---|---|---|---|---|
| *1 : Brightness at 5V (cd/m²) | | | | | |

### Comparative Example 1

An organic EL element was prepared in a similar manner to Example 1, except that the exemplary compound 47 was replaced with a known compound, i.e., the following compound (E). The element showed emission, when a DC voltage was applied thereto under dry environment.

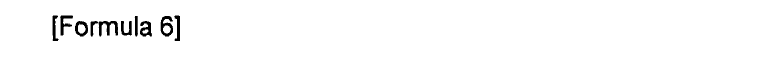

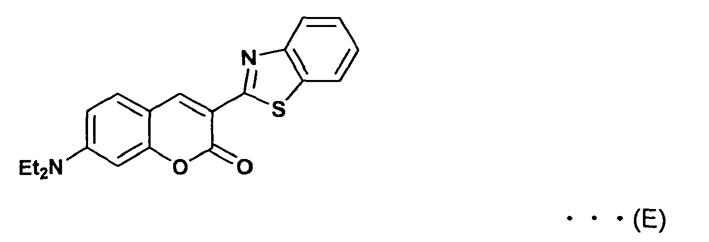

### Comparative Example 2

An organic EL element was prepared in a similar manner to Example 6, except that the exemplary compound 16 was replaced with a known compound, the following compound (F). The element showed emission, when a DC voltage was applied thereto under dry environment.

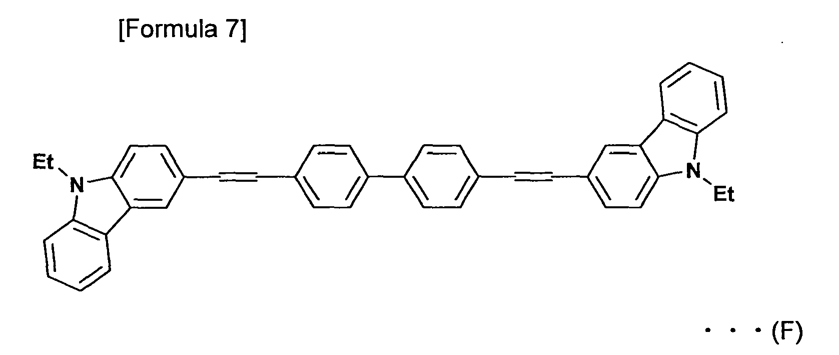

The maximum brightness and the brightness at an operating voltage of 5 V, and the half lifetime under constant-current operation at an emission brightness of 1000 cd/m² of the elements prepared in Comparative Examples 1 and 2 are shown in Table 4. The half lifetime is a relative value, with respect to 100 of the half life of the luminescence brightness of the organic EL element prepared in Example 1.

### [Table 4]

**Table 4**

| Comp.Ex. | Compound | Max. brightness (cd/m²) | Brightness (cd/m²)*¹ | Half lifetime (relative value) | Emission color |
|---|---|---|---|---|---|
| 1 | (E) | 1600 | 40 | 40 | Green |
| 2 | (F) | 2100 | 3 5 | 55 | Blue |

| | | | | | |
|---|---|---|---|---|---|
| *1 : Brightness at 5V (cd/m²) | | | | | |

### Example 11

A glass substrate carrying a transparent ITO electrode (anode) having a thickness of 200 nm was ultrasonicated in a neutral detergent solution, in acetone, and then in ethanol. The substrate was dried by using nitrogen gas, purified with UV/ozone, and fixed to a substrate holder of a vapor deposition apparatus, and the vapor deposition chamber was evacuated to 5×10⁻⁶ Torr. First, the compound (A) was deposited on the transparent ITO electrode at a vapor deposition speed of 0.2 nm/sec to a thickness of 10 nm, to give a hole-injecting layer. Then, the compound (B) was vapor-deposited at vapor deposition speed of 0.2 nm/sec to a thickness of 60nm, to give a hole-transporting layer. Then, the exemplary compound 16 and the exemplary compound 47 were codeposited thereon respectively from different vapor deposition sources at a vapor deposition speed 0.2 nm/sec to a thickness of 40 nm (vapor deposition rate: 100:1), to give a light-emitting layer. Then, bis(2-methyl-5-phenyl-8-quinolinolato)phenolatogallium complex (the following compound (G)) was vacuum-deposited thereon, to form an electron-injecting layer having a thickness of 40 nm. Additionally, lithium fluoride was vacuum-deposited thereon to a thickness of 1 nm and then aluminum to a thickness of 200 nm, to form an electrode, thus giving a light-emitting element. The element showed yellow emission and showed a luminescence brightness of 230 (cd/m²) at a DC voltage of 5 V and a maximum luminescence brightness of 5300 (cd/m²).

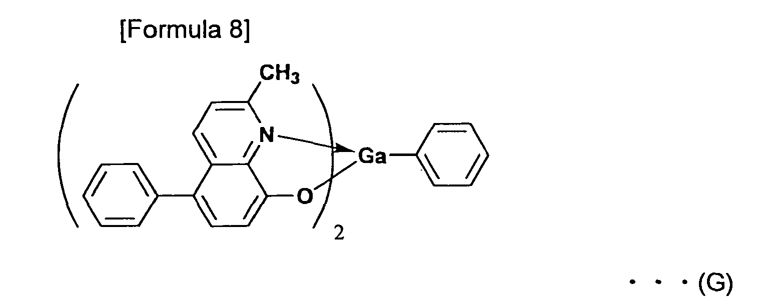

### Examples 12 to 17

An organic EL element was prepared in a similar manner to Example 11, except that the exemplary compound 16 used for the light-emitting layer host material and the exemplary compound 47 used for the light-emitting layer dopant material were respectively replaced with the host material and the dopant material shown in Table 5 (compounds (H), (I), (J) or (K) having the following structure). The element showed blue to red emission, when a DC voltage was applied thereto under dry environment. The maximum brightness, the brightness at an operating voltage of 5 V, and the half lifetime under constant-current operation at an emission brightness of 1000 cd/m² of each element are shown in Table 5.

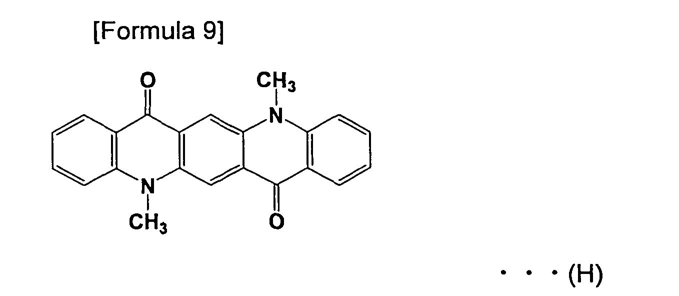

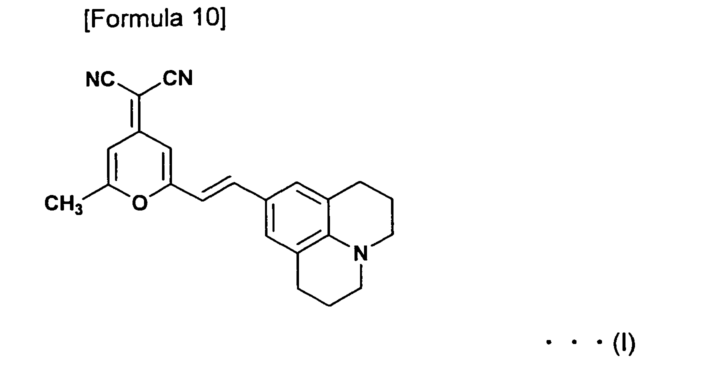

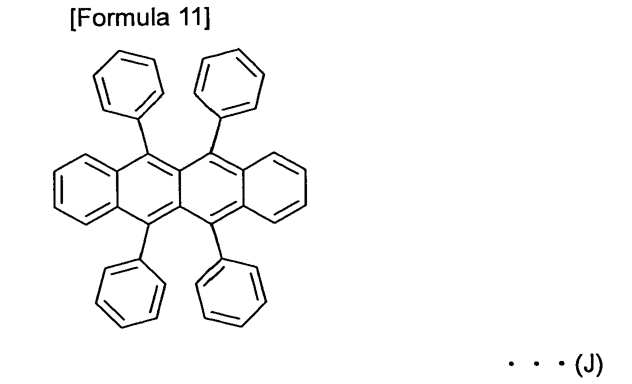

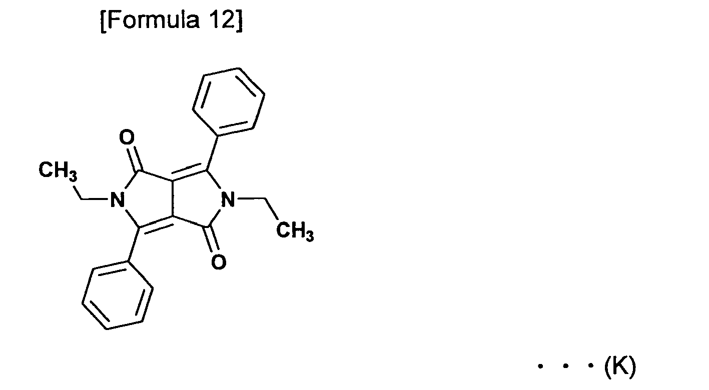

### [Table 5]

**Table 5**

| Ex. | Host material | Dopant material | Max. brightness (cd/m²) | Brightness (cd/m²)*¹ | Emission color |
|---|---|---|---|---|---|
| 11 | 16 | 47 | 5300 | 120 | Yellow |
| 12 | 16 | Compound(E) | 3900 | 130 | Green |
| 13 | 16 | Compound(F) | 2600 | 120 | Blue |
| 14 | 16 | Compound(G) | 4800 | 140 | Green |
| 15 | 47 | Compound(H) | 1500 | 120 | Red |
| 16 | 47 | Compound(I) | 2400 | 170 | Orange |
| 17 | 47 | Compound(K) | 6000 | 190 | Orange yellow |

| | | | | | |
|---|---|---|---|---|---|
| *1 : Brightness at 5V (cd /m²) | | | | | |

### Example 18

A glass substrate carrying a transparent ITO electrode (anode) having a thickness of 200 nm was ultrasonicated in a neutral detergent solution, in acetone, and then in ethanol. The substrate was dried by using nitrogen gas, purified with UV/ozone, and fixed to a substrate holder of a vapor deposition apparatus, and the vapor deposition chamber was evacuated to 5×10⁻⁶ Torr. First, the compound (B) was deposited on the transparent ITO electrode at a vapor deposition speed of 0.2 nm/sec to a thickness of 60 nm, to give a hole-transporting layer. Then, the compound (C) and the compound (G) were codeposited thereon respectively from different vapor deposition sources at a vapor deposition speed 0.2 nm/sec to a thickness of 60 nm (vapor deposition rate: 100:1), to give a light-emitting layer. Then, the exemplary compound 3 was vapor-deposited at a vapor deposition speed of 0.2 nm/sec to a thickness of 40 nm, to give an electron-transporting layer. Additionally, magnesium and silver were codeposited thereon at a vapor deposition speed of 0.2 nm/sec to a thickness of 200 nm (weight ratio: 10:1), to form a cathode, thus giving an organic EL element. Vapor deposition was carried out consecutively while the vapor deposition chamber was kept reduced pressure.

### Examples 19 and 20

An organic EL element was prepared in a similar manner to Example 18, except that the exemplary compound 3 was replaced with exemplary compound 17 or 65. Each element showed green emission, when a DC voltage was applied thereto under dry environment. The maximum brightness, the brightness at an operating voltage of 5 V, and the half lifetime under constant-current operation at an emission brightness of 1000 cd/m² of each element are shown in Table 6. The half lifetime is a relative value, with respect to 100 of the half life of the luminescence brightness of the organic EL element prepared in Example 18.

### [Table 6]

**Table 6**

| Ex. | Compound | Max. brightness (cd/m²) | Brightness (cd/m²)*¹ | Half lifetime (relative value) |
|---|---|---|---|---|
| 18 | 3 | 2000 | 120 | 100 |
| 19 | 17 | 4200 | 180 | 150 |
| 20 | 65 | 3900 | 220 | 140 |

| | | | | |
|---|---|---|---|---|
| *1 : Brightness at 5V (cd/m²) | | | | |

### Comparative Examples 3 and 4

An organic EL element was prepared in a similar manner to Example 18, except that the exemplary compound 3 was replaced with the compound (C) or the following compound (L). The element showed green emission, when a DC voltage was applied thereto under dry environment. The maximum brightness, the brightness at an operating voltage of 5 V, and the half lifetime under constant-current operation at an emission brightness of 1000 cd/m² of each element are shown in Table 7. The half lifetime is a relative value, with respect to 100 of the half life of the luminescence brightness of the organic EL element prepared in Example 18.

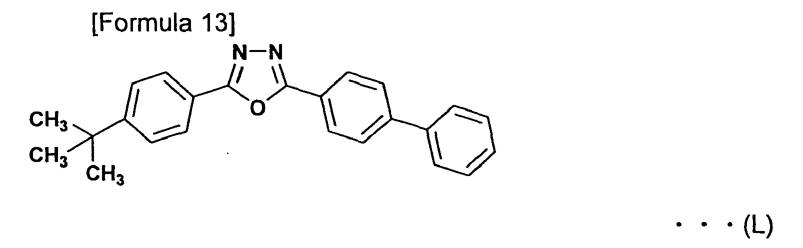

### [Table 7]

**Table 7**

| Comp. Ex. | Compound | Max. brightness (cd/m²) | Brightness (cd/m²)*¹ | Half lifetime (relative value) |
|---|---|---|---|---|
| 3 | (C) | 1600 | 40 | 49 |
| 4 | (L) | 2300 | 20 | 23 |

| | | | | |
|---|---|---|---|---|
| *1 : Brightness at 5V (cd/m²) | | | | |

### Example 21

A solution of the exemplary compound 60, the compound (A), the compound (J), and a polycarbonate resin (Teijin Chemicals Ltd.: Panlite K-1300) at a rate of 5:15:15:65% by weight dissolved in tetrahydrofuran was spin-coated on a cleaned glass plate carrying an ITO electrode, to give a light-emitting layer having a film thickness of 100 nm. The film obtained then was very stable and did not show phenomena such as aggregation and crystallization. An electrode having a thickness of 150 nm was formed thereon with an alloy of magnesium and silver at a rate of 10:1, to give an organic phosphorescent light-emitting element. As for the emission characteristics of the element, the luminescence brightness at a DC voltage of 10 V was 160 (cd/m²), the maximum luminescence brightness was 4100 (cd/m²), and the emission color was green.

### Example 22

A glass substrate carrying a transparent ITO electrode (anode) having a thickness of 200 nm was ultrasonicated in a neutral detergent solution, in acetone, and then in ethanol. The substrate was dried by using nitrogen gas, purified with UV/ozone, and fixed to a substrate holder of a vapor deposition apparatus, and the vapor deposition chamber was evacuated to 5×10⁻⁶ Torr. First, N,N'-di(naphthalen-1-yl)-N,N'-diphenyl-benzidine (compound (B)) was vapor-deposited on the transparent ITO electrode at a vapor deposition speed of 0.2 nm/sec to a thickness of 70 nm, to give a hole-injecting and transporting layer. Then, 9,10-di-(2-naphthyl)anthracene (β-ADN) (the following compound (M)) and the exemplary compound 52 were codeposited thereon from different vapor deposition sources at a vapor deposition speed of 0.2 nm/sec to a thickness of 40 nm (vapor deposition rate: 100:2), to give a light-emitting layer. Then, tris(8-quinolinolato)aluminum (compound (C)) was vapor-deposited at a vapor deposition speed of 0.2 nm/sec and to a thickness of 20 nm, to give an electron-transporting layer. Additionally, lithium fluoride was vapor-deposited thereon to a thickness of 1 nm and additionally aluminum to a thickness of 100 nm, to form an electrode, finally giving a light-emitting element.

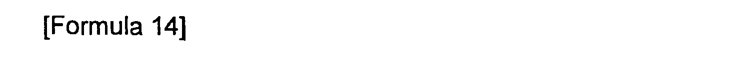

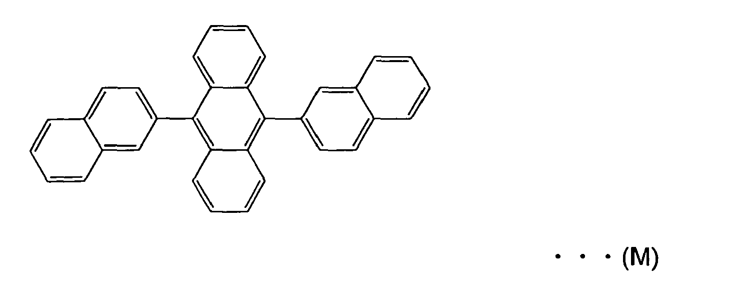

### Example 23 to Example 32

An organic EL element was prepared in a similar manner to Example 22, except that the exemplary compound 52 was replaced with the compound shown in Table 8. The element showed blue to green emission, when a DC voltage was applied thereto under dry environment. The maximum brightness, the brightness at an operating voltage of 5 V and the emission color of these elements are summarized in Table 8.

### [Table 8]

**Table 8**

| Ex. | Compound | Max. brightness (cd/m²) | Brightness (cd/m²)*¹ | Emission color |
|---|---|---|---|---|
| 22 | 52 | 9800 | 190 | Green |
| 23 | 9 | 7000 | 160 | Blue |
| 24 | 10 | 7800 | 180 | Blue |
| 25 | 16 | 8100 | 170 | Blue |
| 26 | 30 | 9400 | 210 | Bluish green |
| 27 | 32 | 1030 | 240 | Bluish green |
| 28 | 35 | 8300 | 180 | Blue |
| 2 9 | 51 | 1010 | 220 | Green |
| 30 | 70 | 790 0 | 170 | Blue |
| 31 | 74 | 7900 | 200 | Blue |
| 32 | 80 | 7400 | 180 | Bluish green |

| | | | | |
|---|---|---|---|---|
| *1 : Brightness at 5V (cd/m²) | | | | |

### Example 33

A glass substrate carrying a transparent ITO electrode (anode) having a thickness of 200 nm was ultrasonicated in a neutral detergent solution, in acetone, and then in ethanol. The substrate was dried by using nitrogen gas, purified with UV/ozone, and fixed to a substrate holder of a vapor deposition apparatus, and the vapor deposition chamber was evacuated to 5×10⁻⁶ Torr. First, N,N'-di(naphthalen-1-yl)-N,N'-diphenyl-benzidine (compound (B)) was vapor-deposited on the transparent ITO electrode at a vapor deposition speed of 0.2 nm/sec to a thickness of 50 nm, to give a hole-injecting and transporting layer. Then, tris(8-quinolinolato)aluminum (compound (C)) was vapor-deposited thereon at a vapor deposition speed of 0.2 nm/sec to a thickness of 30 nm, to give a light-emitting layer. Then, the exemplary compound (93) was vapor-deposited at a vapor deposition speed of 0.2 nm/sec to a thickness of 30 nm, to give an electron-transporting layer. Lithium fluoride was vacuum-deposited thereon to a thickness of 0.8 nm and additionally aluminum to a thickness 100 nm, to form an electrode, finally giving a light-emitting element.

### Examples 34 to 43

An organic EL element was prepared in a similar manner to Example 33, except that the exemplary compound 93 was replaced with the compound shown in Table 9. The element showed yellowish green emission, when a DC voltage was applied thereto under dry environment. The maximum brightness, the brightness at an operating voltage of 5 V, and the half lifetime under constant-current operation at an emission brightness of 1000 cd/m² of each element are shown in Table 9. The half lifetime is a relative value, with respect to 100 of the half life of the luminescence brightness of the organic EL element prepared in Example 33.

### [Table 9]

**Table 9**

| Ex. | Compound | Max. brightness (cd/m²) | Brightness (cd/m²)*¹ | Half lifetime (relative value) |
|---|---|---|---|---|
| 33 | 93 | 8600 | 250 | 100 |
| 34 | 9 | 5100 | 140 | 86 |
| 35 | 17 | 8100 | 230 | 105 |
| 36 | 18 | 7600 | 190 | 110 |
| 37 | 35 | 6200 | 200 | 93 |
| 38 | 51 | 5800 | 110 | 78 |
| 39 | 66 | 9100 | 240 | 107 |
| 40 | 67 | 7400 | 150 | 83 |
| 41 | 68 | 8300 | 180 | 98 |
| 42 | 87 | 9600 | 210 | 99 |
| 43 | 103 | 1010 | 170 | 87 |

| | | | | |
|---|---|---|---|---|
| *1 : Brightness at 5V (cd/m²) | | | | |

### Comparative Examples 5 and 6

An organic EL element was prepared in a similar manner to Example 33, except that the exemplary compound 93 was replaced with the compound (C) or (L). The element showed yellowish green emission, when a DC voltage was applied thereto under dry environment. The maximum brightness, the brightness at an operating voltage of 5 V, and the half lifetime under constant-current operation at an emission brightness of 1000 cd/m² of each element are shown in Table 10.

### [Table 10]

**Table 10**

| Ex. | Compound | Max. brightness (cd/m²) | Brightness (cd/m²)*¹ | Half lifetime (relative value) |
|---|---|---|---|---|
| 5 | C | 6100 | 60 | 51 |
| 6 | L | 6700 | 100 | 64 |

| | | | | |
|---|---|---|---|---|
| *1 : Brightness at 5V (cd/m²) | | | | |

As obvious from the results in Examples described above, the organic EL elements according to the present invention have improved luminescence brightness when operated at low voltage and also have elongated lifetime.

## Claims

1. A material for organic electroluminescent elements, comprising a compound represented by the following General Formula (1): 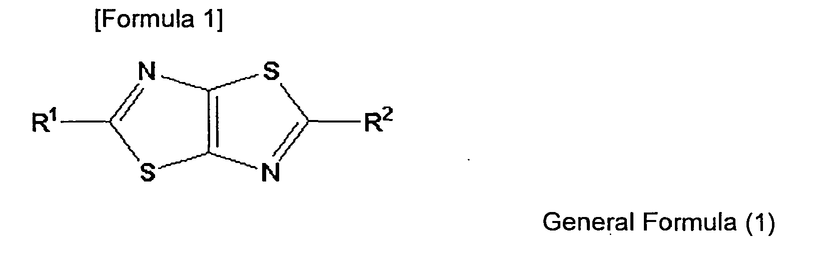 wherein, R¹ and R² each independently represent a substituted or unsubstituted alkyl group, a substituted or unsubstituted aryl group or a substituted or unsubstituted heterocyclic group.

2. The material for organic electroluminescent elements according to Claim 1, wherein R¹ and R² each independently represent a substituted or unsubstituted aryl group having rings of 6 to 30 carbon atoms or a substituted or unsubstituted heterocyclic group having rings of 3 to 30 carbon atoms and 1 to 10 heteroatoms.

3. The material for organic electroluminescent elements according to Claim 1 or 2, wherein the material is a material for the light-emitting layers of organic electroluminescent elements.

4. The material for organic electroluminescent elements according to Claim 1 or 2, wherein the material is a light-emitting material for organic electroluminescent elements.

5. The material for organic electroluminescent elements according to Claim 1 or 2, wherein the material is an electron-injecting/transporting material for organic electroluminescent elements.

6. An organic electroluminescent element, which is obtained by using the material for organic electroluminescent elements according to any one of Claims 1 to 5.
